Europäisches Patentamt

European Patent Office

Office européen des brevets

(11) Veröffentlichungsnummer : **0 058 867**
**B1**

(12) **EUROPÄISCHE PATENTSCHRIFT**

(45) Veröffentlichungstag der Patentschrift :
10.09.86

(51) Int. Cl.⁴ : **A 61 F   2/28**

(21) Anmeldenummer : 82100904.0

(22) Anmeldetag : 09.02.82

(54) **Verfahren zur Herstellung einer Trikalziumphosphat-Knochenkeramik zur Verwendung als Knochenimplantat, insbesondere zur Auffüllung von Hohlräumen oder zur Fixierung von Knochenbruchstücken nach Frakturen und hiernach hergestellter Trikalziumphosphat-Keramikformkörper.**

(30) Priorität : 20.02.81 DE 3106445
03.07.81 DE 3126273
20.08.81 DE 3133015
20.08.81 DE 3133016

(43) Veröffentlichungstag der Anmeldung :
01.09.82 Patentblatt 82/35

(45) Bekanntmachung des Hinweises auf die Patenterteilung : 10.09.86 Patentblatt 86/37

(84) Benannte Vertragsstaaten :
AT BE CH FR GB IT LI NL SE

(56) Entgegenhaltungen :
EP-A- 0 003 979
DE-A- 2 725 665
DE-A- 2 756 256
PHILIPS TECHNISCHE RUNDSCHAU, Band 37 Nr. 9/10, 1977/78, Eindhoven J.G.J. PEELEN et al. "Geisinterter Hydroxylapatit als Biokeramik", Seiten 255 bis 257
HAGERS "Handbuch der pharmazeutischen Praxis", 4. Ausgabe, Band 7 "Arzneiformen und Hilfsstoffe", Teil A "Arzneiformen" 1971, SPRINGER-VERLAG, Berlin, Heidelberg, New york, Seiten 732 bis 735, 760 bis 762

(73) Patentinhaber : **mundipharma GmbH**
**Mundipharma Strasse 2**
**D-6250 Limburg (Lahn) 1 (DE)**

(72) Erfinder : **Eitenmüller, Jürgen, Dr.**
**Römerstrasse 27**
**D-5024 Brauweiler (DE)**
Erfinder : **Rackur, Helmut, Dr.**
**Veilchenstrasse 80**
**D-7537 Remchingen/Singen (DE)**
Erfinder : **Wimmer, Walter**
**Blumenröderstrasse 68**
**D-6250 Limburg/Lahn (DE)**
Erfinder : **Weiss, Marija, Dr.**
**Mundipharma Strasse 2**
**D-6250 Limburg/Lahn (DE)**

(74) Vertreter : **Eisenführ & Speiser**
**Martinistrasse 24**
**D-2800 Bremen 1 (DE)**

**Beschreibung**

Die Erfindung betrifft ein Verfahren zum Herstellen eines mit einem Wirkstoff imprägnierten und eine Depotwirkung aufweisenden gebrannten Tricalciumphospat-Knochenimplantats, welches aus einem gebrannten Formkörper gebildet wird, dem Wirkstoffe zugesetzt werden, und welches ein filmbildendes Polymerisat zur verzögerten Wirkstoffreigabe aufweist, insbesondere zum Auffüllen von Hohlräumen oder zum Fixieren von Knochenbruchstücken nach Frakturen. Sie betrifft weiterhin ein nach diesem Verfahren hergestelltes Tricalciumphosphat-Knochenimplantat zur Auffüllung von Knochen-Hohlräumen oder zum Fixieren von Knochenbruchstücken nach Frakturen.

Knochenimplantate aus Tricalcium-Keramik als Knochenersatz, kombiniert mit Antibiotika als Medikament-Depot vor Ort sind bekannt (Bild der Wissenschaft 10, 1979, 115, 126).

Zum Einzementieren von Implantaten und zur Verankerung von Gelenk-Endoprothesen o. dgl. sind Knochenzemente in verschiedenen Zusammensetzungen bekannt geworden. Derartige Knochenzemente werden beispielsweise durch Vermischen von Vorprodukten aus pulverförmigen Methylmethacrylat-Homo- oder -Copolymerisaten, geeigneten flüssigen Monomeren, meist Methylmethacrylat, einem Katalysator-System und ggf. Röntgenkontrastmitteln zum Erkennen des Zementes im Körper zunächst in Form eines plastischen Teigs erhalten, der dann in den Körper eingebracht wird und durch Polymerisation des Monomers aushärtet.

Nach der DE-A-22 29 702 ist ein Knochenzement aus Polymethylmethacrylat und einem Monomer-Gemisch aus Methylmethacrylat und Methacrylsäureestern höherer Alkohole und einem aus Dibenzoylperoxid und Dimethyl-p-toluidin bestehenden Katalysator-System bekannt.

Es ist ferner bekannt, dem Knochenzement Antibiotika zur Prophylaxe von Infektionen an der Grenzfläche zwischen Knochenzement und Knochen zuzusetzen. Nach der DE-A-20 22 117 werden einem derartigen Knochenzement als Antibiotika Penicillin, Gentamicin und Tetracyclin zugegeben. Aus dem dann ausgehärteten Knochenzement wird das Antibiotikum zunächst in relativ hoher, seine bakterizide bzw. bakteriostatische Wirkung sicherstellender Konzentration freigesetzt. Danach tritt ein Abfall der Konzentration ein, wobei letzlich eine niedrigere Freisetzungsquote über einen längeren Zeitraum verhältnismäßig konstant bleibt. Auch wenn dann noch eine gewisse Langzeit- bzw. Depotwirkung gegeben ist, so ist doch die wirksame Antibiotika-Konzentration sehr gering. Durch Zugabe größerer Mengen an Antibiotika wird wohl eine Erhöhung der wirksamen Konzentration erreicht, die jedoch nur begrenzt bestehen bleibt, da durch einen erhöhten Antibiotika-Zusatz die mechanische Festigkeit des ausgehärteten Knochenzements ungünstig beeinflußt wird.

Nach der DE-B-25 11 122 ist ein Vorprodukt für die Zubereitung von Knochenzement bekannt, das neben einer Gentamicinverbindung entweder pulverförmige Copolymerisate aus Methylmethacrylat und Methylacrylat oder monomeres Methylmethacrylat als Hauptbestandteil aufweist und Gentamicin-Hydrochlorid und/oder Gentamicin-Hydrobromid oder ein Gemisch von Gentamicin-Sulfat mit Natriumchlorid, Kaliumchlorid, Natriumbromid und/oder Kaliumbromid enthält, wodurch ein Vorprodukt zur Zubereitung von Knochenzement erhalten wird, aus dem Wohl das Antibiotikum in höherer Konzentration freigesetzt wird, jedoch bleibt die Wirksamkeit der Antibiotika nicht über einen langen Zeitraum erhalten.

Zur Verringerung von Komplikation beim Einheilen enossaler Halbimplantate ist aus der DE-A-27 56 256 ein ein Polysaccharid enthaltendes Hilfsmittel zum Bedecken und/oder Ausfüllen von Knochengewege bekannt, welches einen sterilen Abschluß zwischen Wundfläche und dem Implantat schaffen soll.

In der Zeitschrift PHILIPS TECHNISCHE RUNDSCHAU, Band 37, Nr. 9/10, 1977/78 ist auf den Seiten 255/257 die Eingung von Calciumphosphat für Implantationszwecke erläutert, und es sind dort verschiedene Möglichkeiten zum Erzeugen bestimmter Porositäten beim Sintern der Knochenimplantate beschrieben. Derartige Implantate stellen einen Ersatz für zerstörte Knochen dar. Nach der Implantation beginnt das Knochengewebe in die Poren des gesinterten Implantates hineinzuwachsen. Mit zunehmenden Heilungsprozeß wird das implantierte Material dadurch mehr und mehr durch neue Knochensubstanz ersetzt.

In dieser Publikation ist ausgeführt, daß dem Calciumphosphat zur Erzeugung einer bestimmten Macro- und Microporosität vor dem Sintern eine gewisse Menge Wasserstoffperoxid zugesetzt wird und daß die gewählte Menge in Verbindung mit der Erwärmungsgeschwindigkeit einen Einfluß auf die sich einstellende Macroporosität hat, während die ebenfalls gewünschte Microporosität durch andere Parameter beeinflußt wird.

Die Verwendung von Hydroxylapatit in Form eines gesinterten Formkörpers ist auch aus der DE-A-27 25 665 bekannt. Dort weist der gesinterte Formkörper eine Einlage aus faserigem Material auf, um das herum das Hydroxylapatit mit einem Additiv, beispielsweise $AlF_3$ angeordnet ist.

Die zuletzt angesprochenen bekannten Implantate weisen keine der oben angesprochenen, aus anderen Publikationen bekannten Wirkstoffe zur Prophylaxe von Infektionen auf.

Demgegenüber beschreibt jedoch die EP-A1-0 003 979 ein implantierbares Pharmaka-Depot nebst Herstellungsverfahren unter Verwendung von Tricalciumphosphat und einem infektionshemmenden Wirkstoff. Darüber hinaus offenbart diese·Publikation, daß die Abgabe-Kinetik des Wirkstoffes mittels eines Hilfsstoffes zu steuern ist.

Gemäß einem Vorschlag dieser Publikation besteht, das bekannte Pharmaka-Depot aus Calciumphosphatpulver, das mit dem Wirkstoff und dem Hilfsstoff zu einer pastösen Masse vermischt und anschließend unter Anwendung von Wärme zu Tabletten verpresst wird, die das Implantat bilden. Ein Sintern der zunächst pastösen Mischung ist nicht vorstellbar, weil die erforderlichen Sinter-Temperaturen sowohl den Wirkstoff als auch den Hilfsstoff zerstören würden. Derartigen Tabletten fehlt nicht nur die Festigkeit gesinterter Tricalciumphosphat-Formkörper, sie bieten auch den Osteoblasten nicht die notwendige Tragfläche für deren Anlagerung. Da schließlich durch das Einmischen auch des Hilfsstoffes davon auszugehen ist, daß dieser sowohl die Wirkstoffpartikel als auch die Calciumphosphatpulver-Partikeln umgibt, muß angenommen werden, daß eine recht große Menge des Hilfsstoffes pro Volumeneinheit der Tablette eingesetzt werden muß, so daß die Mengen an Pulver und Wirkstoff entsprechend verkleinert sind.

Gemäß einem zweiten Vorschlag der EP-Al-0 003 979 wird das Tricalciumphosphat demgegenüber in Form einer gesinterten Matrix verwendet, wodurch die zuvor angesprochenen Festigkeits- und Anlagerungs-Probleme vermieden werden. Zur Bildung der Porosität schlägt diese Publikation vor, den Calciumphosphatkörper rasch und gesteuert aus seiner Sintertemperatur auf Zimmertemperatur abzukühlen, wodurch man den dort bevorzugten Porenanteil von etwa 10 bis 25 % erhält. Wirkstoff und Hilfsstoff werden anschließend in das poröse Gefüge mit Hilfe von Lösungsmitteln eingebracht. Hierdurch und ggf. zusätzlich vorher wird der Wirkstoff mit dem Hilfsstoff enkapsuliert, so daß der Hilfsstoff den Wirkstoff auch innerhalb des porösen Gefüges umgibt.

Auf diese Weise wird erreicht, daß die Wirkstoff-Abgabe während des beginnenden Einheilprozesses verzögert abläuft.

Demgegenüber liegt der Erfindung die Aufgabe zugrunde, ein Tricalciumphosphat-Knochenimplantat der genannten Art anzugeben, bei dem die Wirkstoffabgabe in wirksamer Konzentration über längere Zeiträume hinweg als bisher exakt steuerbar ist.

Das diese Aufgabe lösende Verfahren zum Herstellen eines mit einem Wirkstoff imprägnierten und eine Depotwirkung aufweisenden gebrannten Tricalciumphosphat-Knochenimplantats, welches aus einem gebrannten Formkörper gebildet wird, dem Wirkstoffe zugesetzt werden, und welches ein filmbildendes Polymerisat zur verzögerten Wirkstoffreigabe aufweist, insbesondere zum Auffüllen von Hohlräumen oder zum Fixieren von Knochenbruchstücken nach Frakturen besteht erfindungsgemäß darin, daß dem Tricalciumphosphat vor dem Brennvorgang anorganische und/oder organische Micro- oder Macroporen bildende Substanzen, wie Wasserstoffperoxid, Zucker, Ammoniumbicardonat und/oder Ammoniumcarbaminat, Calciumhydrogenphosphat zur Bildung definierter Porengrößen zugegeben, und die so erhaltene Masse geformt und gebrannt wird, daß dann der so erhaltene Keramik-Formkörper mit einem Wirkstoff, wie einem Desinfektionsmittel, einem Antiseptikum, einem Chemotherapeutikum oder einem Breitbandmicrobizid, wie Polyvinylpyrrolidon-Jod imprägniert wird und daß der fertig imprägnierte Keramik-Formkörper anschließend mit einem Polymer, insbesondere Polydextran in vorgegebener Schichtdicke überzogen wird.

Der Vorteil dieser Lösung besteht darin, daß durch die Art der Porenerzeugung und durch die vom Hilfsstoff unabhängige Einbringung des Wirkstoffes zunächst eine optimale Wirkstoffmenge in dem gebrannten Formkörper untergebracht wird und daß man ohne Rücksicht auf die Wirkstoffmenge die Dicke der Hilfsstoff-Schicht frei bestimmen kann, so daß derartige Implantate entsprechend der jeweiligen Wahl der Schichtdicken kurze, mittlere oder lange Wirkstoff-Abgabezeiten aufweisen.

Als porenbildende Substanz wird bei dem erfindungsgemäßen Verfahren bevorzugt 1 % bis 3 % Wasserstoffperoxid zugegeben.

Bevorzugt wird weiterhin, daß dem Tricalciumphosphat vor dem Brennvorgang zusätzlich eine Fluoridverbindung mit Elementen der Alkalimetall- und/oder Erdalkalimetallgruppe, wie $CaF_2$, NaF, $MgF_2$ zugesetzt wird.

Von Vorteil ist es schließlich, daß der Keramik-Formkörper mit einer Wirkstofflösung bis zu einem Wirkstoffgehalt imprägniert wird, dessen auf seine Trockensubstanz bezogenes Gewicht bis zu 45 % des Keramikausgangsgewichtes beträgt.

Das gebrannte Tricalciumphosphat-Knochenimplantat kann bevorzugt von mehreren unterschiedlichen Schichten eines Polymers überzogen sein, wobei einzelne Schichtenihrerseits mit einem Wirkstoff imprägniert sind.

Nach dem erfindungsgemäßen Verfahren hergestellte Tricalciumphosphat-Knochenimplantate werden bevorzugt zur Auffüllung von Knochen-Hohlräumen oder zum Fixieren von Knochenbruchstücken nach Frakturen verwendet.

Zur Herstellung des Keramik-Formkörpers werden Tricalciumphosphatpulver und die gewählten Zusätze zur Steuerung der Micro- und Macroporosität, insbesondere also die erwähnte Menge Wasserstoffperoxid homogen vermischt und dann verformt. Das Tricalciumphosphatpulver kann mit den Zusätzen granuliert werden. Auch besteht die Möglichkeit, das Tricalciumphosphatpulver zusammen mit den Zusätzen nach dem Schlickerguß-Verfahren zu verformen. Anschließend erfolgt das Brennen bei Temperaturen um 1 000 °C, wobei auch darunterliegende und darüberliegende Temperaturen eingesetzt werden können.

Es sei darauf hingewiesen, daß die dem Tricalciumphosphat-Pulver vor dem Brennen bevorzugt zugesetzte Fluoridverbindung der besseren Resorption der Keramik durch das Knochengewebe sowie der Anregung des Knochenaufbaus dient. Das beispielsweise vorgeschlagene Calciumfluorid unterbricht die kristalline Struktur des Tricalciumphosphates, wodurch eine schnellere Resorption der Keramik erreicht wird.

Von den verschiedenen bereits erwähnten Wirkstoffen, mit denen der Keramik-Formkörper imprägniert werden kann ist Polyvinylpyrrolidon-Jod-Lösung bzw. -Komplex mit 10 % verfügbarem Jod besonders hervorzuheben. Dieser Wirkstoff, der in verschiedenen Molekulargewichten verwendbar ist, hat sich in Verbindung mit Tricalcium-phosphat-Knochenimplantaten als hoch wirksam herausgestellt.

Die Erfindung wird anhand der nachfolgenden Beispiele erläutert :

Beispiel 1

Handelsübliches Tricalciumphosphat mit einer Korngröße von etwa 1 bis 2 μ wird durch Zusatz von Wasser und einem entsprechenden Porenbildner auf einem Tellergranuliergerät granuliert. Soweit Resorptionsbeschleuniger hinzugeben werden sollen, wird dem Ausgangsmaterial ein entsprechender Resorptionsbeschleuniger, wie z. B. Calciumfluorid zugegeben. Die erhaltenen Granulen sind stabil genug, um vermittels Sieben in Fraktionen verschiedener Größe aufgetrennt zu werden. Das fertige Granulat wird anschließend gebrannt Die Brenndauer beträgt etwa 1 Stunde bei einer Temperatur von etwa 1 000 ºC, wobei diese Temperatur auch niedriger oder höher gewählt sein kann. Die erhaltenen Granulen weisen eine ausreichende mechanische Festigkeit auf und sind in der Lage, ausreichende Mengen eines Wirkstoffes, wie Desinfektionsmittel, Antiseptikum, Chemotherapeutikum oder Breitbandmikrobizid, wie Polyvinylpyrrolidon-Jod-Lösung bzw. -Komplex, aufzunehmen.

Ein so gewonnenes Granulat wird mit einer Lösung eines entsprechenden Wirkstoffes, wie Desinfektionsmittel, Antiseptikum, Chemotherapeutikum oder Breitbandmikrobizid, wie Polyvinylpyrrolidon-Jod-Lösung bzw. -Komplex, imprägniert und anschließend getrocknet. Bei einer entsprechenden Porosität ist das Granulat in der Lage einen Gehalt an Wirkstofftrockensubstanz bis zu 45 % des Keramikausgangsgewichtes aufzunehmen.

Das mit einem Wirkstoff, wie Desinfektionsmittel, Antiseptikum, Chemotherapeutikum oder Breitbandmikrobizid, wie Polyvinylpyrrolidon-Jod-Lösung bzw. -Komplex, imprägnierte Granulat wird anschließend in an sich bekannter Weise dragiert und mit mindestens einer Schicht aus einem Polymer-Überzug versehen, die eine genau definierte Schichtdicke zur Freigabe des Wirkstoffes aufweist.

Beispiel 2

Handelsübliches Tricalciumphosphat mit einer Korngröße von etwa 1 bis 2 μ wird durch Zusatz einer geeigneten Granulierflüssigkeit und eines entsprechenden Porenbildners sowie einer resorptionsfördernden Substanz, z. B. Calciumfluorid, nach einem aus der Pharmazeutik bekannten Granulierverfahren, z. B. Wirbelschicht-Sprühgranulation granuliert. Das erhaltene getrocknete Granulat wird auf einer geeigneten Tablettiermaschine zu Tablettenformlingen verpresst. Die fertigen Tabletten werden anschließend gebrannt. Die Brenndauer beträgt etwa 1 Stunde bei einer Temperatur von etwa 1 000 ºC, wobei diese Temperatur niedriger oder höher gewählt werden kann. Durch die Wahl der Brenntemperatur kann die mechanische Festigkeit der Tabletten bestimmt werden. Die gebrannten Tabletten sind in der Lage, ausreichende Mengen des Wirkstoffes, wie Desinfektionsmittel, Antiseptikum, Chemotherapeutikum oder Breitbandmikrobizid, wie Polyvinylpyrrolidon-Jod-Lösung bzw. -Komplex aufzunehmen.

Die gemäß Beispiel 4 gewonnenen Tabletten werden mit einer Lösung eines entsprechenden Wirkstoffes, wie Antiseptikum oder Chemotherapeutikum oder Breitbandmikrobizid, imprägniert und anschließend getrocknet. Bei einer entsprechenden Porosität sind die Tabletten in der Lage, einen Gehalt an Wirkstofftrockensubstanz bis 45 % des Keramikausgangsgewichtes aufzunehmen.

Die mit einem Wirkstoff, wie Antiseptikum oder Chemotherapeutikum oder Breitbandmikrobizid, imprägnierten Tabletten werden anschließend in an sich bekannter Weise dragiert und mit mindestens einer Schicht aus einem Polymer versehen, die eine genau definierte Schichtdicke zur Freigabe des Antiseptikums oder Chemotherapeutikums oder Breitbandmikrobizids aufweist.

Beispiel 3

Handelsübliches Tricalciumphosphat mit einer Korngröße von etwa 1 bis 2 μ wird mit einem entsprechenden Porenbildner sowie einer resorptionsfördernden Substanz, wie z. B. Calciumfluorid, vermischt und durch Zusatz einer geeigneten Flüssikeit angeteigt. Diese Materialmischung wird in Formen beliebiger Größe eingebracht und anschließend gebrannt. Die Brenndauer beträgt etwa 1 Stunde bei einer Temperatur von etwa 1 000 ºC, wobei diese Temperatur auch niedriger oder höher gewählt werden kann. Durch die Wahl der Brenntemperatur kann die mechanische Festigkeit der Formstücke bestimmt werden. Diese Formstücke sind in der Lage, ausreichende Mengen eines Wirkstoffes, wie Desinfektionsmittel, Antiseptikum, Chemotherapeutikum oder Breitbandmikrobizid, wie Polyvinylpyrrolidon-Jod-Lösung bzw. -Komplex, aufzunehmen.

Die so gewonnenen Formkörper werden mit dem Wirkstoff, wie Antiseptikum oder Chemotherapeutikum oder Breitbandmikrobizid, imprägniert und anschliessend getrocknet. Bei ei-

7         **0 058 867**         8

ner entsprechenden Porosität sind die Formstücke in der Lage, einen Gehalt an Wirkstofftrockensubstanz bis 45 % des Keramikausgangsgewichtes aufzunehmen.

Als Breitbandmikrobizid wird z. B. eines Polyvinylpyrrolidon-Jod-Lösung bzw. -Komplex als Wirkstoff verwendet, bei dem 1 g etwa 10 % verfügbares Jod enthält. Auch Polyvinylpyrrolidon-Jod mit den verschiedensten Molekulargewichten kann verwendet werden.

## Patentansprüche

1. Verfahren zum Herstellen eines mit einem Wirkstoff imprägnierten und eine Depotwirkung aufweisenden gebrannten Tricalciumphosphat-Knochenimplantats, welches aus einem gebrannten Formkörper gebildet wird, dem Wirkstoffe zugesetzt werden, und welches ein filmbildendes Polymerisat zur verzögerten Wirkstoffreigabe aufweist, insbesondere zum Auffüllen von Hohlräumen oder zum Fixieren von Knochenbruchstücken nach Frakturen, dadurch gekennzeichnet, daß dem Tricalciumphosphat vor dem Brennvorgang anorganische und/oder organische Micro- oder Macroporen bildende Substanzen, wie Wasserstoffperoxid, Zucker, Ammoniumbicarbonat und/oder Ammoniumcarbaminat, Calciumhydrogenphosphat zur Bildung definierter Porengrößen zugegeben, und die so erhaltene Masse geformt und gebrannt wird, daß dann der so erhaltene Keramik-Formkörper mit einem Wirkstoff, wie einem Desinfektionsmittel, einem Antiseptikum, einem Chemotherapeutikum oder einem Breitbandmicrobizid, wie Polyvinylpyrrolidon-Jod imprägniert wird und daß der fertig imprägnierte Keramik-Formkörper anschließend mit einem Polymer, insbesondere Polydextran in vorgegebener Schichtdicke überzogen wird.

2. Verfahren nach Anspruch 1, dadurch gekennzeichnet, daß als porenbildende Substanz 1 % bis 3 % Wasserstoffperoxid zugegeben wird.

3. Verfahren nach Anspruch 1 oder 2, dadurch gekennzeichnet, daß dem Tricalciumphosphat vor dem Brennvorgang zusätzlich eine Fluoridverbindung mit Elementen der Alkalimetall- und/oder Erdalkalimetallgruppe, wie $CaF_2$, NaF, $MgF_2$ zugesetzt wird.

4. Verfahren nach Anspruch 1 bis 3, dadurch gekennzeichnet, daß der Keramik-Formkörper mit einer Wirkstofflösung bis zu einem Wirkstoffgehalt imprägniert wird, dessen auf seine Trockensubstanz bezogenes Gewicht bis zu 45 % des Keramikausgangsgewichtes beträgt.

5. Gebranntes Tricalciumphosphat-Knochenimplantat, hergestellt nach dem Verfahren gemäß einem der Ansprüche 1 bis 4.

6. Implantat nach Anspruch 5, dadurch gekennzeichnet, daß es von mehreren unterschiedlichen Schichten eines Polymers überzogen ist und daß einzelne Schichten ihrerseits mit einem Wirkstoff imprägniert sind.

7. Implantat nach einem der Ansprüche 5 oder 6, enthaltend eine Fluoridverbindung mit den Elementen der Alkalimetall- und/oder Erdalkalimetallgruppe, wie $CaF_2$, NaF, $MgF_2$.

8. Implantat nach einem der Ansprüche 5 bis 7, enthaltend ein Breitbandmicrobizid bzw. Antiseptikum, insbesondere Polyvinylpyrrolidon-Jod als Wirkstoff.

9. Implantat nach einem der Ansprüche 5 bis 8, enthaltend ein Chemotherapeutikum bzw. Antibiotikum, wie Penicillin, Cycloserin, Bacitracin, Nystatin, Amphotericin, Gentamicin, Novobiocin, Erythromycin, Momycin, Streptomycin als Wirkstoff.

10. Implantat nach einem der Ansprüche 5 bis 9, mit einem Chemotherapeutikum wie Sulfonamid als Wirkstoff.

## Claims

1. Process for the production of a fired tricalcium phosphate bone implant which is impregnated with an active substance and has a depot effect, which implant is formed of a fired moulded body to which active substances are added and which comprises a film-forming polymer for the delayed release of active substances, more especially for filling cavities or for fixing bone fragments after fractures, characterised in that the tricalcium phosphate, prior to the firing operation, has added thereto, inorganic and/or organic substances which form micropores or macropores, such as hydrogen peroxide, sugar, ammonium bicarbonate and/or ammonium carbaminate, calcium hydrogen phosphate, so as to form defined pore sizes, and the mass as thus obtained is moulded and fired, that then the ceramic shaped ceramic element as thus obtained is impregnated with an active substance, such as a disinfecting agent, an antiseptic agent, a chemotherapeutic agent or a broad-band microbicide, such as polyvinyl pyrrolidone iodine, and that the prepared ceramic shaped element is thereafter coated with a polymer, more especially polydextrane, in a prescribed layer thickness.

2. Process according to claim 1, characterised in that 1 % to 3 % of hydrogen peroxide is added as pore-forming substance.

3. Process according to claim 1 or 2, characterised in that the tricalcium phosphate, prior to the firing operation, has additionlly added thereto a fluoride compound with elements of the alkali metal and/or alkaline-earth metal group, such as $CaF_2$. NaF, $MgF_2$.

4. Process according to claims 1 to 3, characterised in that the ceramic shaped element or moulding is impregnated with a solution of active substance to a content thereof of which its weight, related to its dry substance, is up to 45 % of the initial weight of ceramic.

5. Fired tricalcium phosphate bone implant, produced by the process according to one of the claims 1 to 4.

6. Implant according to claim 5, characterised in that it is coated with a plurality of different layers of a polymer and that individual layers are in their turn impregnated with an active substance.

7. Implant according to one of the claims 5 or 6, containing a fluoride compound with the elements of the alkali metal and/or alkaline earth metal group, such as CaF$_2$, NaF, MgF$_2$.

8. Implant according to one of claims 5 to 7, containing a wide-band microbicide or antiseptic, more especially polyvinyl pyrrolidone iodine, as active substance.

9. Implant according to one of the claims 5 to 8, containing a chemotherapeuticc or antibiotic, such as penicillin, cycloserine, bacitracine, nystatine, amphotericine, gentamicine, novobiocine, erythromycine, momycine, streptomycine, as active substance.

10. Implant according to one of claims 5 to 9, with a chemotherapeutic agent, such as sulphonamide, as active substance.

## Revendications

1. Procédé pour la fabrication d'un implant osseux à base de phosphate tricalcique imprégné avec une matière active et présentant une action de dépôt, cet implant étant constitué d'un corps moulé cuit, auquel la matière active est ajoutée et qui comporte un polymère formant un film pour la libération retardée de la matière active, notamment pour le remplissage de cavités ou pour la fixation de fragments osseux après fracture, caractérisé en ce qu'on ajoute du phosphate tricalcique avant l'opération de cuisson des substances anorganiques et/ou organiques, formant des micropores ou des macropores, telles que peroxyde d'hydrogène, sucre, bicarbonate d'ammonium et/ou carbamate d'ammonium, hydrophosphate de calcium pour former des pores de grosseur déterminée, et on moule et on cuit la masse ainsi obtenue, en ce qu'on imprègne ensuite le corps moulé en céramique ainsi obtenu avec une matière active telle qu'un agent de désinfection, un antiseptique, une substance chimiothérapeutique ou un microbicide à large spectre d'action tel que l'iode-polyvinyle-pyrrolidine et en ce qu'on munit ensuite le corps moulé en céramique imprégné et terminé d'un revêtement en polymère, notamment polydextrane, avec une épaisseur de couche prédéterminée.

2. Procédé selon la revendication 1, caractérisé en ce qu'on ajoute en tant que substance de formation de pores 1 % à 3 % de peroxyde d'hydrogène.

3. Procédé selon la revendication 1 ou la revendication 2, caractérisé en ce qu'on ajoute en supplément au phosphate tricalcique avant l'opération de cuisson un composé fluoré contenant des élément du groupe des métaux alcalins et/ou des métaux alcalino-terreux, tel que CaF$_2$, NaF, MgF$_2$.

4. Procédé selon les revendications 1 à 3, caractérisé en ce qu'on imprègne le corps moulé en céramique avec une solution de matière active jusqu'à une teneur en matière active qui, rapportée au poids de substance sèche, va jusqu'à 45 % du poids de céramique initial.

5. Implant osseux cuit à base de phosphate tricalcique, fabriqué d'après le procédé selon l'une des revendications 1 à 4.

6. Implant selon la revendication 5, caractérisé en ce qu'il est revêtu de plusieurs couches différentes d'un polymère et en ce que des couches individuelles sont à leur tour imprégnées avec une matière active.

7. Implant selon l'une des revendications 5 ou 6, contenant un composé fluoré avec des éléments du groupe des métaux alcalins et/ou des métaux alcalino-terreux, tel que CaF$_2$, NaF, MgF$_2$.

8. Implant selon l'une des revendications 5 à 7, contenant un microbicide à large spectre d'action ou un antiseptique, notamment l'iode-polyvinyle-pyrrolidine, en tant que matière active.

9. Implant selon l'une des revendications 5 à 8, contenant une substance chimiothérapeutique ou un antibiotique, tels que la pénicilline, la cyclosérine, la bacitracine, la nystatine, l'amphotéricine, la gentamicine, la novobiocine, l'érythronycine, la momycine, la streptomycine, en tant que matière active.

10. Implant selon l'une des revendications 5 à 9, avec une substance chimiothérapeutique telle que la sulfonamide en tant que matière active.